(19) Europäisches Patentamt
European Patent Office
Office européen des brevets

(11) **EP 4 242 253 B1**

(12) **EUROPEAN PATENT SPECIFICATION**

(45) Date of publication and mention
of the grant of the patent:
**30.04.2025 Bulletin 2025/18**

(21) Application number: **22818593.0**

(22) Date of filing: **31.08.2022**

(51) International Patent Classification (IPC):
*C08L 71/02* (2006.01)      *C08G 65/12* (2006.01)
*C08G 65/26* (2006.01)      *C08G 65/30* (2006.01)
*A61K 47/10* (2017.01)      *A61K 9/20* (2006.01)

(52) Cooperative Patent Classification (CPC):
**C08G 65/2609; A61K 9/2031; A61K 47/10;
C08G 65/12; C08G 65/266; C08G 65/30;
C08L 71/02**

(86) International application number:
**PCT/JP2022/032816**

(87) International publication number:
**WO 2023/145119 (03.08.2023 Gazette 2023/31)**

(54) **POLYALKYLENE OXIDE PARTICLES, USE THEREOF, AND PHARMACEUTICAL COMPOSITION COMPRISING THE PARTICLES**

POLYALKYLENOXIDPARTIKEL, VERWENDUNG DER PARTIKEL UND PHARMAZEUTISCHE ZUSAMMENSETZUNG ENTHALTEND DIE PARTIKEL

PARTICULES D'OXYDE DE POLYALKYLÈNE, LEUR UTILISATION ET COMPOSITION PHARMACEUTIQUE LES COMPRENANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.01.2022   JP 2022013784**

(43) Date of publication of application:
**13.09.2023 Bulletin 2023/37**

(73) Proprietor: **Sumitomo Seika Chemicals Co., Ltd.
Kako-gun, Hyogo 675-0145 (JP)**

(72) Inventors:
• **SAINOU, Naoki
Himeji-shi, Hyogo 672-8076 (JP)**
• **SUKA, Keita
Himeji-shi, Hyogo 672-8076 (JP)**

(74) Representative: **Vossius & Partner
Patentanwälte Rechtsanwälte mbB
Siebertstraße 3
81675 München (DE)**

(56) References cited:
**WO-A1-2012/165198      WO-A1-2012/165199**

**Description**

Technical Field

The present invention relates to polyalkylene oxide particles, a pharmaceutical composition, a preparation composition, and a preparation.

Background Art

Conventionally, polyalkylene oxide particles typified by polyethylene oxide are known to be used as binders for pharmaceutical preparations etc. For example, PTL 1 discloses polyalkylene oxide particles whose particle size distribution is controlled in a specific range, and which can be applied to a binder for pharmaceutical preparations.

Citation List

Patent Literature

PTL 1: WO2012/165198

Summary of Invention

Technical Problem

In recent years, preparations are required to have low performance variation. For example, there is a demand for the development of preparations that have the function of suppressing the variation in the dissolution of drugs etc. From this point of view, it is also important to develop polyalkylene oxide particles that can reduce performance variation in preparations. In this respect, for example, the stability of various physical properties of polyalkylene oxide particles is maintained by controlling the aqueous solution viscosity as a representative physical property value. Such polyalkylene particles are applied to preparations to thereby stabilize the performance of the preparations.

However, as a result of examination, the present inventors found that the aqueous solution viscosity of polyalkylene oxide particles having a particle size distribution varies depending on each particle size, and that as the difference in aqueous solution viscosity between particle sizes is larger, the performance of preparations is more likely to vary, and the dissolution of preparations is particularly more likely to fluctuate. In particular, the inventors found the problem that if the uniformity of polyalkylene oxide particles is disturbed by segregation or breakdown of uniformity due to vibration or the like during transfer of the polyalkylene oxide particles, variation in the performance (particularly dissolution) of preparations using such particles is particularly increased.

The present invention was made in view of the above, and its object is to provide polyalkylene oxide particles that can reduce the variation in the degree of dissolution of preparations, and that can make variation less likely to occur in the degree of dissolution of preparations even if the uniformity of the polyalkylene oxide particles is disturbed by vibration or the like. Another object of the present invention is to provide a pharmaceutical composition comprising the polyalkylene oxide particles, and a preparation composition comprising the pharmaceutical composition.

Solution to Problem

The present inventors conducted extensive research to achieve the above object, and consequently found that the above object can be achieved by polyalkylene oxide particles having specific aqueous solution viscosity characteristics. Thus, the present invention has been completed.

Specifically, the present invention includes, for example, the main subjects described in the following items.

Item 1.

Polyalkylene oxide particles that satisfy a viscosity ratio A (%) of 66% or more and 150% or less, wherein the viscosity ratio A is represented by the following formula (1):

$$A = (A1/A2) \times 100 \quad (1)$$

wherein A1 is the 1 mass% aqueous solution viscosity (mPa·s) of particles having a particle size of 150 $\mu$m or more, and A2 is the 1 mass% aqueous solution viscosity (mPa·s) of particles having a **particle size of less than 150 $\mu$m, and wherein**

**the viscosity and the particle size are measured as indicated in the description below.**

Item 2.

The polyalkylene oxide particles according to Item 1, wherein when X parts by mass of the polyalkylene oxide particles are sieved in sequence by a sieve with a mesh opening of 300 $\mu$m, a sieve with a mesh opening of 250 $\mu$m, a sieve with a mesh opening of 180 $\mu$m, a sieve with a mesh opening of 150 $\mu$m, a sieve with a mesh opening of 106 $\mu$m, a sieve with a mesh opening of 75 $\mu$m, and a saucer, the mass ratios of polyalkylene oxide particles remaining on the sieves and the saucer are all 5 mass% or more based on X.

Item 3.

Use of the polyalkylene oxide particles according to Item 1 or 2 for a preparation.

Item 4.

A pharmaceutical composition comprising the polyalkylene oxide particles according to any one of Items 1 to 3.

Item 5.

A preparation composition comprising the pharmaceutical composition according to Item 4.

Item 6.

The preparation composition according to Item 5, which comprises 20 mass% or more of the polyalkylene oxide particles.

Item 7.

A preparation comprising the preparation composition according to Item 5 or 6.

Advantageous Effects of Invention

The polyalkylene oxide particles of the present invention can reduce the variation in the degree of dissolution of preparations, and can make variation less likely to occur in the degree of dissolution of preparations even if the uniformity of the polyalkylene oxide particles is disturbed by vibration or the like.

Description of Embodiments

Embodiments of the present invention are described in detail below. In the present specification, the terms "comprising" and "containing" include the concepts of "comprising," "containing," "consisting essentially of," and "consisting of."
In the numerical range described in stages in the present specification, the upper or lower limit of the numerical range at one stage can be optionally combined with the upper or lower limit of the numerical range at another stage. In the numerical range described in the present specification, the upper or lower limit of the numerical range may be replaced with a value shown in the Examples or a value that can be uniquely derived from the Examples. Further, in the present specification, the numerical values connected by "to" mean the numerical range including the numerical values before and after "to" as the lower limit and the upper limit.

1. Preparation Polyalkylene Oxide Particles

The preparation polyalkylene oxide particles of the present invention (hereinafter simply referred to as "the polyalkylene oxide particles of the present invention" or "the polyalkylene oxide particles") satisfy a viscosity ratio A (%) of 66% or more and 150% or less, wherein the viscosity ratio A is represented by the following formula (1):

$$A = (A1/A2) \times 100 \quad (1)$$

wherein A1 is the 1 mass% aqueous solution viscosity (mPa·s) of particles having a particle size of 150 $\mu$m or more, and A2 is the 1 mass% aqueous solution viscosity (mPa·s) of particles having a particle size of less than 150 μm.

For example, when used as a binder (excipient) for forming preparations in a dosage form such as tablets, the polyalkylene oxide particles can reduce the variation in the degree of dissolution of the preparations. That is, when the polyalkylene oxide particles of the present invention are used to prepare multiple preparations, the variation in the degree of dissolution is low between the preparations. In addition, even if the uniformity of the polyalkylene oxide particles is disturbed by vibration or the like, the polyalkylene oxide particles of the present invention can make variation less likely to occur in the degree of dissolution of the preparations. Therefore, the polyalkylene oxide particles of the present invention can be preferably used to form preparations.

The polyalkylene oxide particles have a particle form, and the type thereof is not particularly limited as long as the viscosity ratio A (%) is 66% or more and 150% or less.

In the polyalkylene oxide particles, the type of polyalkylene oxide is not particularly limited, and examples include a wide range of known polyalkylene oxides.

In the polyalkylene oxide, the number of carbon atoms in the alkylene moiety is preferably 2 or more and 4 or less, for example. Because the effects of the present invention can be easily exhibited, it is particularly preferable that the alkylene moiety has 2 carbon atoms; that is, the polyalkylene oxide is preferably polyethylene oxide. Specific examples of polyalkylene oxides other than polyethylene oxide include polypropylene oxide, polybutylene oxide, ethylene oxide/propylene oxide copolymers, ethylene oxide/butylene oxide copolymers, and the like.

The polyalkylene oxide is generally a homopolymer, but is not limited thereto, and may be a copolymer. When the polyalkylene oxide is a copolymer, the polyalkylene oxide has, for example, two or more structural units with alkylene moieties having different number of carbon atoms.

The alkylene moiety of the polyalkylene oxide particles preferably contains at least an ethylene oxide unit, in terms of ease of production and ease of reduction of the variation in the degree of dissolution of preparations. That is, in the polyalkylene oxide particles, the polyalkylene oxide is preferably polyethylene oxide, or a copolymer of an ethylene oxide unit and other units (e.g., an ethylene oxide/propylene oxide copolymer or an ethylene oxide/butylene oxide copolymer, mentioned above).

The polyalkylene oxide particles may contain one type of polyalkylene oxide, or two or more types of polyalkylene oxides.

The polyalkylene oxide particles have a particle size distribution, and the particle size distribution is not particularly limited as long as the viscosity ratio A satisfies the above range.

For example, when X parts by mass of the polyalkylene oxide particles are sieved using a sieve with a mesh opening of 300 μm, a sieve with a mesh opening of 250 μm, a sieve with a mesh opening of 180 μm, a sieve with a mesh opening of 150 μm, a sieve with a mesh opening of 106 μm, a sieve with a mesh opening of 75 μm, and a saucer in this order, the mass ratios P (%) of polyalkylene oxide particles remaining on the sieves and the saucer are all preferably 5 mass% or more based on X. In this case, when applied to preparations, the polyalkylene oxide particles are likely to reduce the variation in the degree of dissolution of the preparations.

In the above sieving, the mass ratio P of polyalkylene oxide particles remaining on the sieve with a mesh opening of 300 μm is preferably 0 to 30 mass%, and more preferably 0 to 20 mass%. In the sieving, the mass ratio P of polyalkylene oxide particles remaining on the sieve with a mesh opening of 250 μm is preferably 0 to 30 mass%, and more preferably 0 to 20 mass%. In the sieving, the mass ratio P of polyalkylene oxide particles remaining on the sieve with a mesh opening of 180 μm is preferably 5 to 50 mass%, and more preferably 10 to 40 mass%. In the sieving, the mass ratio P of polyalkylene oxide particles remaining on the sieve with a mesh opening of 150 μm is preferably 5 to 70 mass%, and more preferably 5 to 30 mass%. In the sieving, the mass ratio P of polyalkylene oxide particles remaining on the sieve with a mesh opening of 106 μm is preferably 5 to 70 mass%, and more preferably 5 to 30 mass%. In the sieving, the mass ratio P of polyalkylene oxide particles remaining on the sieve with a mesh opening of 75 μm is preferably 5 to 70 mass%, and more preferably 5 to 30 mass%. In the sieving, the mass ratio P of polyalkylene oxide particles remaining on the saucer is preferably 5 to 70 mass%, and more preferably 11 to 50 mass%.

The content ratio of particles having a particle size of 150 μm or more in the polyalkylene oxide particles of the present invention is preferably 30 to 70 mass%, and more preferably 40 to 60 mass%. Further, the content ratio of particles having a particle size of 300 μm or more in the polyalkylene oxide particles of the present invention is preferably less than 20 mass%.

The content ratio of polyalkylene oxide particles having a particle size of 150 μm or more in the polyalkylene oxide particles can be calculated by classifying the polyalkylene oxide particles by a sieve with a mesh opening of 150 μm (JIS Z 8801-1 standard sieve). Specifically, the polyalkylene oxide particles are classified by a sieve with a mesh opening of 150 μm, the mass of polyalkylene oxide particles remaining on the sieve is measured, and the ratio thereof based on the total mass of the polyalkylene oxide particles used in classification is calculated to thereby determine the content ratio of polyalkylene oxide particles having a particle size of 150 μm or more. As is clear from this explanation, the phrase "polyalkylene oxide particles having a particle size of 150 μm or more" refers to, after the polyalkylene oxide particles are classified by a sieve with a mesh opening of 150 μm, particles remaining on the sieve.

The content ratio of polyalkylene oxide particles having a particle size of 300 μm or more in the polyalkylene oxide particles can be calculated by classifying the polyalkylene oxide particles by a sieve with a mesh opening of 300 μm (JIS Z 8801-1 standard sieve). Specifically, the polyalkylene oxide particles are classified by a sieve with a mesh opening of 300 μm, the mass of polyalkylene oxide particles remaining on the sieve is measured, and the ratio thereof based on the total mass of the polyalkylene oxide particles used in classification is calculated to thereby determine the content ratio of polyalkylene oxide particles having a particle size of 300 μm or more. As is clear from this explanation, the phrase "polyalkylene oxide particles having a particle size of 300 μm or more" refers to, after the polyalkylene oxide particles are classified by a sieve with a mesh opening of 300 μm, particles remaining on the sieve.

In the polyalkylene oxide particles, as described above, the viscosity ratio A (%) represented by the following formula (1):

$$A = (A1/A2) \times 100 \quad (1)$$

is 66% or more and 150% or less. In the formula, A1 is the 1 mass% aqueous solution viscosity (mPa·s) of particles having a particle size of 150 μm or more, and A2 is the 1 mass% aqueous solution viscosity (mPa·s) of particles having a particle size of less than 150 μm.

In formula (1), the particles having a particle size of 150 μm or more can refer to, when the polyalkylene oxide particles are classified by a sieve with a mesh opening of 150 μm, polyalkylene oxide particles remaining on the sieve. Alternatively, in formula (1), the particles having a particle size of 150 μm or more can refer to, when a sieve with a mesh opening of 300 μm, a sieve with a mesh opening of 250 μm, a sieve with a mesh opening of 180 μm, and a sieve with a mesh opening of 150 μm are stacked in sequence from above, and the polyalkylene oxide particles are put in the uppermost sieve with a mesh opening of 300 μm for classification, polyalkylene oxide particles remaining on each sieve. Further, the particles having a particle size of less than 150 μm refer to, when the polyalkylene oxide particles are classified by a sieve with a mesh opening of 150 μm, polyalkylene oxide particles passing through the sieve.

Because the viscosity ratio A falls within the above range, the polyalkylene oxide particles of the present invention can reduce the variation in the degree of dissolution of preparations; that is, when the polyalkylene oxide particles of the present invention are used to prepare multiple preparations, the variation in the degree of dissolution can be reduced between the preparations. In addition, even if the uniformity of the polyalkylene oxide particles is disturbed by vibration or the like, the variation in the degree of dissolution of the preparations can be suppressed.

The viscosity ratio A is preferably 66% or more, more preferably 71% or more, and even more preferably 80% or more. Further, the viscosity ratio A is preferably 150% or less, more preferably 140% or less, and even more preferably 125% or less.

The 1 mass% aqueous solution viscosity (A1 (mPa·s)) of polyalkylene oxide particles having a particle size of 150 μm or more is 40 to 20000 mPa·s, or the 5 mass% aqueous solution viscosity is preferably 30 to 50000 mPa·s. The 1 mass% aqueous solution viscosity is preferably 1000 mPa·s or more, and more preferably 2000 mPa·s or more, and is more preferably 10000 mPa·s or less, and particularly preferably 7500 mPa·s or less. The 5 mass% aqueous solution viscosity is preferably 30 to 50000 mPa·s, more preferably 40 mPa·s or more, and even more preferably 100 mPa·s or more. The 1 mass% aqueous solution viscosity and 5 mass% aqueous solution viscosity, described later, of the polyalkylene oxide particles can be measured using a rotational viscometer (RV DVII+, produced by Brookfield).

The 1 mass% aqueous solution viscosity (A1 (mPa·s)) of polyalkylene oxide particles having a particle size of less than 150 μm is 40 to 20000 mPa·s, or the 5 mass% aqueous solution viscosity is preferably 30 to 50000 mPa·s. The 1 mass% aqueous solution viscosity is preferably 1000 mPa·s or more, and more preferably 2000 mPa·s or more, and is more preferably 10000 mPa·s or less, and particularly preferably 7500 mPa·s or less. The 5 mass% aqueous solution viscosity is preferably 30 to 50000 mPa·s, more preferably 40 mPa·s or more, and even more preferably 100 mPa·s or more. The 1 mass% aqueous solution viscosity and 5 mass% aqueous solution viscosity, described later, of the polyalkylene oxide particles can be measured using a rotational viscometer (RV DVII+, produced by Brookfield).

The 1 mass% aqueous solution viscosity of the polyalkylene oxide particles of the present invention is 40 to 20000 mPa·s, or the 5 mass% aqueous solution viscosity is preferably 30 to 50000 mPa·s. The 1 mass% aqueous solution viscosity and 5 mass% aqueous solution viscosity as mentioned herein each refer to the aqueous solution viscosity of the entire polyalkylene oxide particles (total particle size). In this case, the variation in the degree of dissolution of preparations can be reduced, and even if the uniformity of the polyalkylene oxide particles is disturbed by vibration or the like, the variation in the degree of dissolution of preparations is more likely to be suppressed.

The 1 mass% aqueous solution viscosity of the polyalkylene oxide particles in the total particle size range (total particle size) is preferably 1000 mPa·s or more, and more preferably 2000 mPa·s or more, and is more preferably 15000 mPa·s or less, and even more preferably 7500 mPa·s or less. Further, the 5 mass% aqueous solution viscosity of the polyalkylene oxide particles (total particle size) is preferably 30 to 50000 mPa·s, more preferably 40 mPa·s or more, and even more preferably 100 mPa·s or more. When the 5 mass% aqueous solution viscosity of the polyalkylene oxide particles (total particle size) is 30 to 50000 mPa·s, the 1 mass% aqueous solution viscosity is preferably less than 40 mPa·s.

The methods for adjusting the viscosity ratio A of polyalkylene oxide particles and the aqueous solution viscosity of the particle size of polyalkylene oxide particles are not particularly limited. For example, known methods can be widely used.

As the method for adjusting the viscosity ratio A of polyalkylene oxide particles, polyalkylene oxide particles that satisfy the above viscosity ratio A can be obtained, for example, by mixing polyalkylene oxide particles sieved by a sieve with a mesh opening of 150 μm and having a particle size of 150 μm or more and a specific 1 mass% aqueous solution viscosity, with polyalkylene oxide particles having a particle size of less than 150 μm and a specific 1 mass% aqueous solution viscosity. For example, the viscosity ratio A can be easily adjusted by mixing polyalkylene oxide particles having a particle size of 150 μm or more and a known 1 mass% aqueous solution viscosity, with polyalkylene oxide particles having a particle size of less than 150 μm.

In another method for adjusting the viscosity ratio A, two or more types of polyalkylene oxide particles having different particle size distributions are prepared, and these particles are mixed at a prescribed ratio. Furthermore, polyalkylene oxide particles that satisfy the viscosity ratio A can be obtained by sieving two or more types of polyalkylene oxide particles having different particle size distributions to produce various particle size groups, and mixing two or more of the particle groups at a prescribed ratio. In an embodiment thereof, for example, two types of polyalkylene oxide particles $P_A$ and $P_B$ having different particle size distributions are prepared. These particles are each sieved in such a manner that a sieve with a mesh opening of 300 μm, a sieve with a mesh opening of 250 μm, a sieve with a mesh opening of 180 μm, a sieve with a mesh opening of 150 μm, a sieve with a mesh opening of 106 μm, a sieve with a mesh opening of 75 μm, and a saucer are stacked in sequence from above, and the polyalkylene oxide particles are put in the uppermost sieve with a mesh opening of 300 μm for sieving. As a result of the sieving operation, polyalkylene oxide particles having 7 particle sizes derived from polyalkylene oxide particles $P_A$ and $P_B$, i.e., a total of 14 polyalkylene oxide particle groups, are obtained. The 1 mass% aqueous solution viscosity of each of the 14 polyalkylene oxide particle groups is measured in the manner described above, and with reference to the measurement results, any two or more of the groups are combined and mixed. Then, the viscosity ratio A of the resulting polyalkylene oxide particles is measured, and polyalkylene oxide particles having a viscosity ratio A of 66% or more and 150% or less can be obtained as the polyalkylene oxide particles of the present invention.

The method for adjusting the 1 mass% aqueous solution viscosity or 5 mass% aqueous solution viscosity of polyalkylene oxide particles is not particularly limited. Examples include methods of adjusting the type of raw material used in the production of polyalkylene oxide particles, the ratio of raw material used, polymerization temperature, polymerization time, the amounts of solvent and chain transfer agent, and other conditions.

The bulk specific gravity (loose) of the polyalkylene oxide particles is preferably 0.15 to 0.60 g/mL. In this case, production efficiency and transport efficiency are excellent, and the formability of the preparation tends to be good. The bulk specific gravity (loose) of the polyalkylene oxide particles is more preferably 0.20 to 0.55 g/mL. In the present invention, the loose bulk density of the polyalkylene oxide particles refers to a value measured according to JIS K6720 4.3.

The mass average molecular weight of the polyalkylene oxide particles is not particularly limited. In terms of easily imparting low friability to the preparation and reducing thermal expansion coefficient, the mass average molecular weight of the polyalkylene oxide particles is preferably 100,000 or more and 15 million or less. The mass average molecular weight of the polyalkylene oxide particles is more preferably 200,000 to 12 million, even more preferably 2 million to 10 million, and particularly preferably 3 million to 8 million. The mass average molecular weight of the polyalkylene oxide particles as mentioned herein refers to a value measured by gel permeation chromatography, and particularly refers to a value calculated from a calibration curve created using a known polyethylene oxide standard sample.

The form of the polyalkylene oxide particles is not particularly limited, and may be, for example, spherical, ellipsoidal, or amorphous.

The viscosity ratio A of the polyalkylene oxide particles satisfies the specific range, and when used as a binder (excipient) for forming preparations in a dosage form such as tablets, the polyalkylene oxide particles can reduce the variation in the degree of dissolution of the preparations. In addition, even if the uniformity of the polyalkylene oxide particles is disturbed by vibration or the like, the polyalkylene oxide particles of the present invention can make variation less likely to occur in the degree of dissolution of preparations.

When used as an excipient for preparations, the polyalkylene oxide particles of the present invention can suppress the variation in the performance of the preparations without changing the composition of the preparations. Therefore, the polyalkylene oxide particles of the present invention are preferable for pharmaceuticals and preparations, and particularly preferable as an excipient for tablets.

2. Method for Producing Polyalkylene Oxide Particles

As the method for producing the polyalkylene oxide particles of the present invention, for example, methods for producing known polyalkylene oxide particles can be widely used.

For example, the polyalkylene oxide particles can be obtained by polymerization reaction of an alkylene oxide in the presence of an alkali or a metal catalyst. Examples of the alkylene oxide used herein include aliphatic alkylene oxides. Specific examples include ethylene oxide, propylene oxide, and butylene oxide; preferably ethylene oxide or propylene oxide; and particularly preferably ethylene oxide. Alkylene oxides can be used singly or in combination of two or more.

The catalyst can be, for example, an alkali catalyst or a metal catalyst. As the metal catalyst, for example, metal catalysts conventionally used in the production of polyalkylene oxide can be widely used. Of these, an organic zinc catalyst is preferred. Organic zinc catalysts can be obtained by known production methods, preferably by reacting organic zinc compounds with aliphatic polyhydric alcohols and monohydric alcohols to form particulate reaction products.

The amount of catalyst used can be the same as in methods for producing known polyalkylene oxide particles. For example, the catalyst can be used in a catalytic amount.

The polymerization reaction of the alkylene oxide can be performed in a solvent. As such solvents, those used in methods for producing known polyalkylene oxides can be widely used. Examples include at least one hydrocarbon solvent selected from the group consisting of 2-methylpentane, n-pentane, n-hexane, n-heptane, isopentane, and cyclohexane; aromatic hydrocarbons, such as benzene, toluene, and xylene; and the like. N-hexane or n-pentane is preferably used because they are easily available industrially, and because they have a boiling point lower than the melting point of the resulting polyalkylene oxide and are easy to remove after the polymerization reaction. The amount of polymerization solvent used is preferably 100 to 10000 parts by mass, more preferably 200 to 2000 parts by mass, and even more preferably 400 to 600 parts by mass, based on 100 parts by mass of alkylene oxide, in terms of removing the heat of polymerization and easily controlling the polymerization reaction.

A $C_{1-5}$ alcohol compound can be used in the polymerization reaction of the alkylene oxide. Examples include $C_{1-5}$ alcohol compounds, such as methanol, ethanol, n-propanol, isopropanol, n-butanol, isobutanol, and t-butanol.

The temperature and other conditions of the polymerization reaction of the alkylene oxide are not particularly limited, and can be the same as known conditions.

## 3. Pharmaceutical Composition

The pharmaceutical composition of the present invention comprises the preparation polyalkylene oxide particles of the present invention described above. Therefore, when used as an excipient for forming preparations in a dosage form such as tablets, the pharmaceutical composition of the present invention can reduce the variation in the degree of dissolution of the preparations. In addition, even if the uniformity of the polyalkylene oxide particles is disturbed by vibration or the like, the pharmaceutical composition of the present invention can make variation less likely to occur in the degree of dissolution of preparations. Therefore, the pharmaceutical composition of the present invention is suitable as a raw material for preparing preparation compositions.

The pharmaceutical composition of the present invention may consist of the polyalkylene oxide particles, or may contain components other than the polyalkylene oxide particles.

## 4. Preparation Composition

The preparation composition of the present invention comprises the pharmaceutical composition of the present invention described above. Specifically, the pharmaceutical composition of the present invention may contain the polyalkylene oxide particles and components other than the polyalkylene oxide particles.

As components other than the polyalkylene oxide particles (hereinafter referred to as other components), for example, various components contained in known preparation compositions can be widely applied. Specific examples of other components include active components, fillers, excipients other than polyalkylene oxide particles, diluents, lubricants, dyes, pigments, osmotic agents, and the like.

The preparation composition of the present invention may contain the polyalkylene oxide particles and silica as a filler. When the preparation composition of the present invention contains the polyalkylene oxide particles and silica, the preparation composition of the present invention preferably contains silica in an amount of 3.0 parts by mass or less based on 100 parts by mass of the polyalkylene oxide particles. This tends to enhance the fluidity etc. of the polyalkylene oxide particles. The content of silica based on 100 parts by mass of the polyalkylene oxide particles is, for example, more preferably 2.0 parts by mass or less, and even more preferably 1.5 parts by mass or less. In terms of easily enhancing the fluidity etc., the content of silica based on 100 parts by mass of the polyalkylene oxide particles is, for example, more preferably 0.1 parts by mass or more.

As silica, for example, known silica can be widely used. Specifically, Aerosil and the like can be used.

The content ratio of the polyalkylene oxide particles in the preparation composition is not particularly limited as long as the effects of the present invention are not impaired. In terms of easily imparting particularly low friability to the preparation, the preparation composition preferably contains the polyalkylene oxide particles in an amount of 20 mass% or more, preferably 30 mass% or more, more preferably 40 mass% or more, and even more preferably 50 mass% or more, based on the total mass of the polyalkylene oxide particles and the other components (or the total mass of the preparation composition). Further, the polyalkylene oxide particles are preferably contained in an amount of 90 mass% or less based on the total mass of the polyalkylene oxide particles and the other components (or the total mass of the preparation composition).

In particular, the preparation composition of the present invention contains the polyalkylene oxide particles, and thus has excellent compression molding properties, and a compression molded article can be easily obtained. The method for obtaining the compression molded article is not particularly limited. For example, known compression molding methods can be widely used.

The method for preparing the preparation composition of the present invention is not particularly limited, and can be the same as, for example, methods for preparing known preparation compositions. For example, the preparation composition can be prepared by mixing polyalkylene oxide particles and a filler (e.g., silica) to obtain, for example, filler-coated polyalkylene oxide particles, and then mixing the polyalkylene oxide particles with various other components at a specific ratio.

The preparation composition of the present invention can be used to prepare various preparations. Since such preparations contain the preparation composition of the present invention, i.e., the polyalkylene oxide particles, the variation in the degree of dissolution of the preparations can be easily reduced.

The preparation of the present invention may contain a compression molded article of the preparation composition. In this case, the preparation can be formed into various dosage forms, including compression molded articles. Examples include tablets.

Examples

The present invention is described in more detail below with reference to Examples; however, the present invention is not limited to these Examples.

Production Example 1

Diethyl zinc was diluted with n-hexane to a concentration of 0.66 mol/L in terms of zinc in a vessel purged with nitrogen. The vessel was then cooled to 10°C, and 1,4-butanediol was added to the vessel under stirring until the concentrations of 1,4-butanediol and ethanol reached 0.59 mol/L and 2.58 mol/L, respectively, with respect to n-hexane. After the completion of the addition, the temperature in the vessel was raised to 30°C, and diethyl zinc was reacted with 1,4-butanediol and ethanol for 1 hour. Next, the temperature was raised to 50°C, and the reaction was performed for 1 hour. The temperature in the vessel was then raised to 80°C, and distillation was performed. After cooling, the reaction liquid in the vessel was diluted with n-hexane so that the concentration of the organic zinc catalyst was 3 mass%, thereby obtaining a dispersion containing the organic zinc catalyst.

Subsequently, in a pressure-resistant vessel purged with nitrogen, an organic zinc catalyst, a $C_{1-5}$ alcohol compound, and n-hexane were added so that the concentration of the organic zinc catalyst in terms of zinc was 0.0022 mol/L with respect to n-hexane and the concentration of t-butanol was 0.0041 mol/L with respect to n-hexane, and they were uniformed dispersed. Then, ethylene oxide was added to a concentration of 3.84 mol/L with respect to hexane, the vessel was sealed, and the mixture was polymerized with stirring in a thermostatic bath at 40°C. After the completion of the polymerization, the produced white product was filtered out and dried at 40°C. The resulting dried particles were mixed with 1 mass% of amorphous silica (Aerosil, produced by Nippon Aerosil Co., Ltd.), and the mixture was transferred to a JIS Z 8801-1 standard sieve (500 $\mu$m), thereby obtaining polyethylene oxide particles $P_A$ passing through the sieve.

Production Example 2

Polyethylene oxide particles $P_B$ were obtained in the same manner as in Production Example 1, except that the concentration of t-butanol with respect to n-hexane was changed to 0.00041 mol/L.

Production Example 3

Classification of Polyethylene Oxide Particles

For polyethylene oxide particles $P_A$ obtained in Production Example 1, as JIS Z 8801-1 standard sieves, a sieve with a mesh opening of 300 $\mu$m, a sieve with a mesh opening of 250 $\mu$m, a sieve with a mesh opening of 180, a sieve with a mesh opening of 150 $\mu$m, a sieve with a mesh opening of 106 $\mu$m, and a sieve with a mesh opening of 75 $\mu$m were stacked in this order on a saucer. The polyethylene oxide particles were placed in the uppermost sieve with a mesh opening of 500 $\mu$m. The sieves were shaken using a rotating-tapping shaker for 20 minutes to classify the polyethylene oxide particles.

After classification, the mass of polyethylene oxide particles remaining on each sieve was measured, and the percentage of each mass relative to the total mass (particle size distribution) was calculated.

Production Example 4

Classification was performed in the same manner as in Production Example 3, except that polyethylene oxide particles $P_B$ obtained in Production Example 2 were used in place of polyethylene oxide particles $P_A$. Then, the mass of polyethylene oxide particles remaining on each sieve was measured, and the percentage of each mass relative to the total mass (particle size distribution) was calculated.

Table 1

| Sample | Mass ratio of particles remaining on each sieve (mass%) | | | | | | |
|---|---|---|---|---|---|---|---|
| | 300 μm | 250 μm | 180 μm | 150 μm | 106 μm | 75 μm | Saucer |
| Polyethylene oxide particles $P_A$ (classification results in Production Example 3) | 4.1 | 2.2 | 24.9 | 12.8 | 11.8 | 6.5 | 37.7 |
| Polyethylene oxide particles $P_B$ (classification results in Production Example 4) | 0.7 | 3.8 | 31.6 | 7.3 | 12.0 | 13.7 | 30.8 |

Table 1 shows the classification results (classification results of polyethylene oxide particles $P_A$ and $P_B$) in Production Examples 3 and 4.

Example 1

In the classification of polyethylene oxide particles $P_A$, 3 g of particles remaining on each sieve and the saucer were each collected (7 types in total). In the classification of polyethylene oxide particles $P_B$, 3 g of particles remaining on each sieve and the saucer were each collected (7 types in total). These 14 types of particles were uniformly mixed to obtain preparation polyethylene oxide particles. The preparation polyethylene oxide particles were classified by a 150-μm sieve (JIS Z 8801-1 standard sieve), and particles on the 150-μm sieve and particles passing through the sieve were collected. The 1% aqueous solution viscosities A1 (mPa·s) and A2 (mPa·s) of each type of particles were measured, and the viscosity ratio A (%) was calculated by the following formula (1):

$$A = (A1/A2) \times 100 \quad (1).$$

Example 2

The viscosity ratio A (%) was calculated in the same manner as in Example 1, except that in the classification of polyethylene oxide particles $P_A$, particles on the 300-μm sieve, particles on the 180-μm sieve, particles on the 150-μm sieve, particles on the 75-μm sieve, and particles remaining on the saucer were collected (6 g each) (5 types in total), in the classification of polyethylene oxide particles $P_B$, particles on the 250-μm sieve and particles on the 106-μm sieve were collected (6 g each) (2 types in total), and these 7 types of particles were uniformly mixed to obtain preparation polyethylene oxide particles.

Example 3

The viscosity ratio A (%) was calculated in the same manner as in Example 1, except that in the classification of polyethylene oxide particles $P_A$, particles on the 180-μm sieve, particles on the 150-μm sieve, particles on the 106-μm sieve, and particles remaining on the saucer were collected (6 g each) (4 types in total), in the classification of polyethylene oxide particles $P_B$, particles on the 300-μm sieve, particles on the 250-μm sieve, and particles on the 75-μm sieve were collected (6 g each) (3 types in total), and these 7 types of particles were uniformly mixed to obtain preparation polyethylene oxide particles.

Comparative Example 1

The viscosity ratio A (%) was calculated in the same manner as in Example 1, except that in the classification of polyethylene oxide particles $P_A$, particles on the 106-$\mu$m sieve, particles on the 75-$\mu$m sieve, and particles remaining on the saucer were collected (6 g each) (3 types in total), in the classification of polyethylene oxide particles $P_B$, particles on the 300-$\mu$m sieve, particles on the 250-$\mu$m sieve, particles on the 180-$\mu$m sieve, and particles on the 150-$\mu$m sieve were collected (6 g each) (4 types in total), and these 7 types of particles were uniformly mixed to obtain preparation polyethylene oxide particles.

Comparative Example 2

The viscosity ratio A (%) was calculated in the same manner as in Example 1, except that in the classification of polyethylene oxide particles $P_A$, particles on the 300-$\mu$m sieve, particles on the 250-$\mu$m sieve, particles on the 180-$\mu$m sieve, and particles on the 150-$\mu$m sieve were collected (6 g each) (4 types), in the classification of polyethylene oxide particles $P_B$, particles on the 106-$\mu$m sieve, particles on the 75-$\mu$m sieve, and particles remaining on the saucer were collected (6 g each) (3 types), and these 7 types of particles were uniformly mixed to obtain preparation polyethylene oxide particles.

Comparative Example 3

The viscosity ratio A (%) was calculated in the same manner as in Example 1, except that in the classification of polyethylene oxide particles $P_A$, particles on the 180-$\mu$m sieve, particles on the 150-$\mu$m sieve, particles on the 106-$\mu$m sieve, particles on the 75-$\mu$m sieve, and particles remaining on the saucer were collected (6 g each) (5 types in total), in the classification of polyethylene oxide particles $P_B$, particles on the 300-$\mu$m sieve and particles on the 250-$\mu$m sieve were collected (6 g each) (2 types in total), and these 7 types of particles were uniformly mixed to obtain preparation polyethylene oxide particles.

Table 2

| Particle size distribution | | | Ex. 1 | | Ex. 2 | | Ex. 3 | | Comp. Ex. 1 | | Comp. Ex. 2 | | Comp. Ex. 3 | |
|---|---|---|---|---|---|---|---|---|---|---|---|---|---|---|
| Particle size | | Ratio (mass%) | Raw material | Viscosity (mPa·s) | Raw material | Viscosity (mPa·s) | Raw material | Viscosity (mPa·s) | Raw material | Viscosity (mPa·s) | Raw material | Viscosity (mPa·s) | Raw material | Viscosity (mPa·s) |
| Particles on 300-μm sieve | Particles having particle size of 150 μm or more | 14.3 | $P_A$:$P_B$ =1:1 | A1= 5140 | $P_A$ | A1= 3780 | $P_B$ | A1= 4940 | $P_B$ | A1= 7960 | $P_A$ | A1= 2620 | $P_B$ | A1= 4940 |
| Particles on 250-μm sieve | | 14.3 | $P_A$:$P_B$ =1:1 | | $P_B$ | | $P_B$ | | $P_B$ | | $P_A$ | | $P_B$ | |
| Particles on 180-μm sieve | | 14.3 | $P_A$:$P_B$ =1:1 | | $P_A$ | | $P_A$ | | $P_B$ | | $P_A$ | | $P_A$ | |
| Particles on 150-μm sieve | | 14.3 | $P_A$:$P_B$ =1:1 | | $P_A$ | | $P_A$ | | $P_B$ | | $P_A$ | | $P_A$ | |
| Particles on 106-μm sieve | Particles having particle size of less than 150 μm | 14.3 | $P_A$:$P_B$ =1:1 | A2= 4780 | $P_B$ | A2= 3960 | $P_A$ | A2= 3820 | $P_A$ | A2= 2160 | $P_B$ | A2= 7340 | $P_A$ | A2= 2160 |
| Particles on 75-μm sieve | | 14.3 | $P_A$:$P_B$ =1:1 | | $P_A$ | | $P_B$ | | $P_A$ | | $P_B$ | | $P_A$ | |
| Particles on saucer | | 14.3 | $P_A$:$P_B$ =1:1 | | $P_A$ | | $P_A$ | | $P_A$ | | $P_B$ | | $P_A$ | |
| Entire particles | | 100 | Entire particles | 4920 | Entire particles | 3860 | Entire particles | 4480 | Entire particles | 5340 | Entire particles | 4400 | Entire particles | 3700 |
| Viscosity ratio A value (A1/A2 value) | | | 108 | | 95 | | 129 | | 369 | | 36 | | 229 | |

Table 2 shows the mixing conditions of the preparation polyethylene oxide particles obtained in the Examples and Comparative Examples, and the calculation results of the viscosity ratio A. Further, Table 2 shows the 1 mass% aqueous solution viscosity A1 (mPa·s) of particles having a particle size of 150 $\mu$m or more, and the 1 mass% aqueous solution viscosity A2 (mPa·s) of particles having a particle size of less than 150 $\mu$m, and also shows the 1 mass% aqueous solution viscosity of the entire particles (mixture of particles having a particle size of 150 $\mu$m or more and particles having a particle size of less than 150 $\mu$m).

From the results of Table 2, all of the preparation polyethylene oxide particles obtained in Examples 1 to 3 had a viscosity ratio A (%) of 66% or more and 150% or less.

The 1 mass% aqueous solution viscosity was measured in the following manner.

1 Mass% Aqueous Solution Viscosity of Polyethylene Oxide

6 g of the polyethylene oxide particles and 125 mL of isopropanol were added to a 1-L beaker, and while stirring at 350 rpm using an impeller, 594 g of ion-exchange water was added, and the mixture was stirred for 1 minute. Then, the stirring speed was changed to 60 rpm, and stirring was further continued for 3 hours, thereby obtaining a 1 mass% aqueous solution of polyethylene oxide. The aqueous solution was maintained at 25°C, the viscosity was measured using a rotational viscometer (RV DVII+, produced by Brookfield; spindle: RV-2, rotation speed: 2 rpm), and this value was taken as the 1 mass% aqueous solution viscosity.

5 Mass% Aqueous Solution Viscosity of Polyethylene Oxide

30 g of the polyethylene oxide particles and 125 mL of isopropanol were added to a 1-L beaker, and while stirring at 350 rpm using an impeller, 570 g of ion-exchange water was added, and the mixture was stirred for 1 minute. Then, the stirring speed was changed to 60 rpm, and stirring was further continued for 3 hours, thereby obtaining a 5 mass% aqueous solution of polyethylene oxide. The aqueous solution was maintained at 25°C, the viscosity was measured using a rotational viscometer (RV DVII+, produced by Brookfield), and this value was taken as the 5 mass% aqueous solution viscosity.

Evaluation of Variation in Degree of Dissolution of Preparations

(1) Shaking Treatment

Assuming the vibration applied during transportation of the preparation polyethylene oxide particles, shaking treatment was performed by the following procedure. 21 g of the preparation polyethylene oxide particles were put into a 100-mL poly beaker and shaken uniformly. The poly beaker was fixed vertically on a shaker (AS-1N, produced by AS ONE Corporation) and shaken at a frequency of 250 rpm for 10 minutes. Approximately 2 g of particles were collected from each of the upper and lower parts of the shaken poly beaker (upper and lower samples, respectively) .

(2) Compression Molding

200 mg of sample collected by shaking treatment was put into a general-purpose autograph mortar (produced by Ichihashi Seiki Co., Ltd., Φ10, R10), and compression-molded using Autograph (AGS-T, produced by Shimadzu Corporation) with a test force of 5 kN at a compression speed of 100 mm/min to obtain a tablet of polyethylene oxide particles alone. The same operation was repeated to produce a total of 3 tablets for the particles collected from the upper part, and to also produce a total of 3 tablets for the particles collected from the lower part. Thus, a total of 6 tablets (i.e., n=6 in total, n=3 for the upper samples, and n=3 for the lower samples) were obtained.

(3) Dissolution Test

Using the tablets of polyethylene oxide particle alone obtained as described above, a dissolution test was performed according to the Japanese Pharmacopoeia (paddle method) (test liquid: ion-exchange water, test temperature: 37.0°C, stirring speed: 200 rpm). In the dissolution test, the tablets taken after stirring for 8 hours were dried at 80°C for 5 hours, and then the dissolution rate of the polyethylene oxide tablets (upper sample: n=3, lower sample: n=3) was calculated based on the following formula:

Dissolution rate (%) = (1-dry tablet mass after dissolution test/tablet mass before dissolution test)×100

Further, from the obtained dissolution rates (%), the average dissolution rate of the total of 6 tablets was calculated.

(4) Calculation of Coefficient of Variation of Dissolution Rate

From the dissolution test results of the tablets (total n=6, breakdown: upper sample: n=3, lower sample: n=3), the average dissolution rate (n=6) of the total of 6 tablets and the standard deviation (n=6) were calculated, and the coefficient of variation was calculated according to the following formula: Coefficient of variation = (standard deviation (n=6)/average dissolution rate (n=6))×100

Table 3

| | Collected area | Number of tests | Tablet mass [mg] | | Dissolution rate | Average dissolution rate (%) | Standard deviation | Coefficient of variation |
|---|---|---|---|---|---|---|---|---|
| | | | Before test | After test | [%] | | | |
| Ex. 1 | Upper | n=1 | 200.1 | 117.8 | 41.1 | 41.2 | 0.3 | 0.8 |
| | | n=2 | 200.1 | 116.5 | 41.8 | | | |
| | | n=3 | 200.2 | 117.8 | 41.2 | | | |
| | Lower | n=1 | 198.8 | 117.3 | 41.0 | | | |
| | | n=2 | 199.6 | 117.5 | 41.1 | | | |
| | | n=3 | 198.8 | 117.8 | 40.7 | | | |
| Ex. 2 | Upper | n=1 | 199.3 | 124.8 | 37.4 | 37.8 | 0.4 | 1.1 |
| | | n=2 | 198.4 | 122.8 | 38.1 | | | |
| | | n=3 | 198.2 | 122.3 | 38.3 | | | |
| | Lower | n=1 | 198.1 | 122.6 | 38.1 | | | |
| | | n=2 | 197.7 | 124 | 37.3 | | | |
| | | n=3 | 197.7 | 123.6 | 37.5 | | | |
| Ex. 3 | Upper | n=1 | 198.6 | 114.8 | 42.2 | 43.6 | 1.3 | 3.0 |
| | | n=2 | 198.8 | 113.8 | 42.8 | | | |
| | | n=3 | 199.1 | 110.3 | 44.6 | | | |
| | Lower | n=1 | 198.7 | 114.7 | 42.3 | | | |
| | | n=2 | 197.4 | 109.7 | 44.4 | | | |
| | | n=3 | 197.7 | 107.6 | 45.6 | | | |
| Comp. Ex. 1 | Upper | n=1 | 199.8 | 107.8 | 46.0 | 43.9 | 4.1 | 9.3 |
| | | n=2 | 199.2 | 103.6 | 48.0 | | | |
| | | n=3 | 199.9 | 102.6 | 48.7 | | | |
| | Lower | n=1 | 199 | 120.8 | 39.3 | | | |
| | | n=2 | 198.4 | 112.5 | 43.3 | | | |
| | | n=3 | 199 | 123.3 | 38.0 | | | |
| Comp. Ex. 2 | Upper | n=1 | 199.7 | 136.3 | 31.7 | 36.4 | 4.0 | 11.1 |
| | | n=2 | 199.9 | 137 | 31.5 | | | |
| | | n=3 | 200.5 | 130 | 35.2 | | | |
| | Lower | n=1 | 199.6 | 123.7 | 38.0 | | | |
| | | n=2 | 200.5 | 122.6 | 38.9 | | | |
| | | n=3 | 200.2 | 114.4 | 42.9 | | | |

(continued)

|  | Collected area | Number of tests | Tablet mass [mg] | | Dissolution rate | Average dissolution rate (%) | Standard deviation | Coefficient of variation |
|---|---|---|---|---|---|---|---|---|
|  |  |  | Before test | After test | [%] |  |  |  |
| Comp. Ex. 3 | Upper | n=1 | 199.4 | 117.3 | 41.2 | 38.3 | 2.8 | 7.3 |
|  |  | n=2 | 199.3 | 121.9 | 38.8 |  |  |  |
|  |  | n=3 | 198.1 | 114.7 | 42.1 |  |  |  |
|  | Lower | n=1 | 198.7 | 122.7 | 38.2 |  |  |  |
|  |  | n=2 | 198.1 | 129.8 | 34.5 |  |  |  |
|  |  | n=3 | 198 | 128.4 | 35.2 |  |  |  |

Table 3 shows the dissolution rates of the tablets obtained using the preparation polyethylene oxide particles produced in the Examples and Comparative Examples (total n=6, upper sample: n=3, lower sample: n=3), average dissolution rate, standard deviation, and coefficient of variation. In Table 3, the terms "Upper" and "Lower" refer to the upper and lower samples, respectively.

The results shown in Table 3 reveal that tablets (preparations) obtained using polyethylene oxide particles that satisfy a viscosity ratio A (%) of 66% or more and 150% or less have a low coefficient of variation. That is, it was revealed that polyethylene oxide particles having a viscosity ratio A in the above range can reduce the variation in the degree of dissolution of preparations. In particular, it was revealed that even if the uniformity of polyethylene oxide particles is disturbed by vibration or the like, the above particles can make variation less likely to occur in the degree of dissolution of preparations.

Therefore, even if the uniformity of the particle size distribution is disturbed by vibration or the like during transfer, it is possible to suppress the variation in quality; thus, it was demonstrated that the polyethylene oxide particles of the present invention greatly contribute to the quality improvement of preparations.

## Claims

1.  Polyalkylene oxide particles that satisfy a viscosity ratio A (%) of 66% or more and 150% or less, wherein the viscosity ratio A is represented by the following formula (1):

$$A = (A1/A2) \times 100 \qquad (1)$$

wherein A1 is the 1 mass% aqueous solution viscosity (mPa·s) of particles having a particle size of 150 $\mu$m or more, and A2 is the 1 mass% aqueous solution viscosity (mPa·s) of particles having a particle size of less than 150 $\mu$m, and
wherein the viscosity and the particle size are measured as indicated in the description.

2.  The polyalkylene oxide particles according to claim 1, wherein when X parts by mass of the polyalkylene oxide particles are sieved in sequence by a sieve with a mesh opening of 300 $\mu$m, a sieve with a mesh opening of 250 $\mu$m, a sieve with a mesh opening of 180 $\mu$m, a sieve with a mesh opening of 150 $\mu$m, a sieve with a mesh opening of 106 $\mu$m, a sieve with a mesh opening of 75 $\mu$m, and a saucer, the mass ratios of polyalkylene oxide particles remaining on the sieves and the saucer are all 5 mass% or more based on X.

3.  Use of the polyalkylene oxide particles according to claim 1 or 2 for a preparation.

4.  A pharmaceutical composition comprising the polyalkylene oxide particles according to claim 1 or 2.

5.  A preparation composition comprising the pharmaceutical composition according to claim 4.

6.  The preparation composition according to claim 5, which comprises 20 mass% or more of the polyalkylene oxide particles.

7.  A preparation comprising the preparation composition according to claim 5 or 6.

**Patentansprüche**

1. Polyalkylenoxidteilchen, die ein Viskositätsverhältnis A (%) von 66% oder mehr und 150% oder weniger erfüllen, wobei das Viskositätsverhältnis A durch die folgende Formel (1) dargestellt ist:

$$A = (A1/A2) \times 100 \qquad (1)$$

wobei A1 die Viskosität (mPa·s) einer 1-massenprozentigen wässrigen Lösung von Teilchen mit einer Teilchengröße von 150 μm oder mehr ist und A2 die Viskosität (mPa·s) einer 1-massenprozentigen wässrigen Lösung von Teilchen mit einer Teilchengröße von weniger als 150 μm ist und
wobei die Viskosität und die Teilchengröße wie in der Beschreibung angegeben gemessen werden.

2. Die Polyalkylenoxidteilchen nach Anspruch 1, wobei, wenn X Massenteile der Polyalkylenoxidteilchen nacheinander durch ein Sieb mit einer Maschenöffnung von 300 μm, ein Sieb mit einer Maschenöffnung von 250 μm, ein Sieb mit einer Maschenöffnung von 180 μm, ein Sieb mit einer Maschenöffnung von 150 μm, ein Sieb mit einer Maschenöffnung von 106 μm, ein Sieb mit einer Maschenöffnung von 75 μm und einen Untersetzer gesiebt werden, die Massenverhältnisse von auf den Sieben und dem Untersetzer verbleibenden Polyalkylenoxidteilchen alle 5 Massen-% oder mehr, bezogen auf X, betragen.

3. Verwendung der Polyalkylenoxidteilchen nach Anspruch 1 oder 2 für eine Zubereitung.

4. Ein Arzneimittel, umfassend die Polyalkylenoxidteilchen nach Anspruch 1 oder 2.

5. Eine Zubereitungszusammensetzung, umfassend das Arzneimittel nach Anspruch 4.

6. Die Zubereitungszusammensetzung nach Anspruch 5, welche 20 Massen-% oder mehr der Polyalkylenoxidteilchen umfasst.

7. Eine Zubereitung, umfassend die Zubereitungszusammensetzung nach Anspruch 5 oder 6.

**Revendications**

1. Particules d'oxyde de polyalkylène qui satisfont un rapport de viscosité A (%) de 66 % ou plus et de 150 % ou moins, dans lesquelles le rapport de viscosité A est représenté par la formule (1) suivante :

$$A = (A1/A2) \times 100 \qquad (1)$$

où A1 est la viscosité en solution aqueuse à 1 % en masse (mPa·s) de particules ayant une taille de particules de 150 μm ou plus, et A2 est la viscosité en solution aqueuse à 1 % en masse (mPa- s) de particules ayant une taille de particules inférieure à 150 μm, et
où la viscosité et la taille de particules sont mesurées comme indiqué dans la description.

2. Particules d'oxyde de polyalkylène selon la revendication 1, dans lesquelles, lorsque X parties en masse des particules d'oxyde de polyalkylène sont tamisées séquentiellement par un tamis ayant une ouverture de maille de 300 μm, un tamis ayant une ouverture de maille de 250 μm, un tamis ayant une ouverture de maille de 180 μm, un tamis ayant une ouverture de maille de 150 μm, un tamis ayant une ouverture de maille de 106 μm, un tamis ayant une ouverture de maille de 75 μm, et une soucoupe, les rapports en masse des particules d'oxyde de polyalkylène restantes sur les tamis et la soucoupe sont tous de 5 % en masse ou plus sur la base de X.

3. Utilisation des particules d'oxyde de polyalkylène selon la revendication 1 ou 2 pour une préparation.

4. Composition pharmaceutique comprenant les particules d'oxyde de polyalkylène selon la revendication 1 ou 2.

5. Composition de préparation comprenant la composition pharmaceutique selon la revendication 4.

6. Composition de préparation selon la revendication 5, qui comprend 20 % en masse ou plus des particules d'oxyde de polyalkylène.

**7.** Préparation comprenant la composition de préparation selon la revendication 5 ou 6.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012165198 A